Europäisches Patentamt

European Patent Office

Office européen des brevets

(1) Publication number: **0 199 992**
**B1**

# EUROPEAN PATENT SPECIFICATION

(45) Date of publication of patent specification: **29.08.90**

(51) Int. Cl.⁵: **A 61 K 37/02,** A 61 K 47/00

(21) Application number: **86104149.9**

(22) Date of filing: **26.03.86**

(54) Adsorption-resistant peptide composition and use of benzalkonium or benzethonium chloride in the preparation thereof.

(30) Priority: **28.03.85 JP 61926/85**

(43) Date of publication of application:
**05.11.86 Bulletin 86/45**

(45) Publication of the grant of the patent:
**29.08.90 Bulletin 90/35**

(84) Designated Contracting States:
**AT BE CH DE FR GB IT LI NL SE**

(56) References cited:
**EP-A-0 095 751**
**FR-A-2 156 901**
**FR-A-2 325 386**
**GB-A-2 127 689**

**Römpps Chemielexikon 1979, Band I, page 559**

(73) Proprietor: **Eisai Co., Ltd.**
**6-10, Koishikawa 4-chome Bunkyo-ku Tokyo 112 (JP)**

(72) Inventor: **Kakimoto, Fumio**
**5-133-3, Mitsuike-cho Unuma**
**Kagamigahara-shi Gifu Pref. (JP)**
Inventor: **Asakawa, Naoki**
**105-2, Nakoida-cho**
**Kagamigahara-shi Gifu Pref. (JP)**
Inventor: **Ishibashi, Yasuo**
**880-86, Nagaraobusa**
**Gifu Pref. (JP)**
Inventor: **Miyake, Yasuo**
**1-17, Aza-Todomegi Hashizume**
**Inuyama-shi Aichi Pref. (JP)**

(74) Representative: **Lehn, Werner, Dipl.-Ing. et al**
**Hoffmann, Eitle & Partner Patentanwälte**
**Arabellastrasse 4**
**D-8000 München 81 (DE)**

**Description**

This invention relates to an adsorption-resistant peptide composition, and more specifically to an adsorption-resistant peptide composition which contains benzalkonium chloride and/or benzethonium chloride as an essential component.

In general, many of peptides have physiological effects as peptide hormones for example. Upon their administration as drugs, their doses are very small, i.e., on the order of several micrograms and these doses must be strictly followed. In other words, there is a special requirement that the above substances, which have been formulated in very small amounts into preparations, must be administered precisely in their formulated amounts.

It has however been well-known that proteins and peptides, which are contained in an aqueous solution, tend to adhere on glass or its analogous material and as a result, their contents are caused to decrease considerably compared with their original amounts in a preparation.

It has also been known that when insulin is injected as an illustrative peptide in an infusion container, a substantial portion of the insulin is instantaneously adsorbed on the glass surface of the container and the adsorption is liable to occur at a lower insulin concentration ["Pharmaceutics", 39, 107—111 (1979)]. Since doses of peptide hormones such as insulin are limited to very small levels by their nature, they are in a state liable to easy adsorption on glass containers. Moreover, the rates of their adsorption loss reach significant levels because their initial contents are low, leading to considerable decreases to their actually-administered amounts.

Even if a peptide is accurately incorporated in a prescribed amount while paying special attention to containers and other equipment to avoid its adsorption upon formulation of a dosable preparation, it will be adsorbed in a significant amount on the glass wall of a syringe container or the glass wall of an infusion container when the preparation is transferred in the syringe cylinder or is injected in the infusion container for its mixed injection along with the infusion fluid upon actual administration of the peptide. As a result, the amount of the peptide will be decreased to a considerable extent.

Plastic syringe cylinders (made of polypropylene), infusion bottles (made of polypropylene or polyethylene), instillator tubes (made of polyvinyl chloride) and the like have found wide-spread commercial utility in recent years. Peptides are therefore more liable to adsorption. With the foregoing in view, the present inventor conducted a research with a view toward developing a method for preventing adsorption of insulin, secretin and other peptides upon their contact to the plastic walls of various containers and equipment. As a result, it was found that the above object can be achieved by adding one or more substances, which are selected from the group consisting of lecithin, ethylene oxide-propylene oxide copolymers, hydroxypropylcellulose, methylcellulose, polyoxyethylene-hardened castor oil, polyethylene glycol sorbitan oleate, methylcyclodextrin and sorbitan fatty acid esters, to a composition which contains insulin, secretin or other peptide. The above finding led to completion of inventions, on which patent applications were filed under Japanese Patent Application Nos. 89807/1982, 186089/1982 and 122421/1983.

GB—A—2 127 689 discloses a composition comprising calcitonin, benzalkonium chloride and a liquid diluent or carrier, whereby the benzalkonium chloride is used as a preservative.

FR—A—2 325 386 deals with the use of benzethonium chloride in the preparation of pharmaceutical compositions for rectal administration.

Thereafter, the present inventor proceed with a further investigation on adsorption of various peptides upon their contact with container walls and prevention of their adsorption. As a result, it has been found that the above object can be achieved by adding benzalkonium chloride and/or benzethonium chloride to an aqueous peptide-containing solution or suspension, leading to completion of the present invention. The invention therefore concerns the use of benzalkonium chloride and/or benzethonium chloride for the inhibition of adsorption of peptides on glass and plastic.

In a further aspect of this invention, there is thus provided an adsorption-resistant peptide composition against glass and plastic comprising benzalkonium chloride and/or benzethonium chloride as an essential component with the exception of a composition comprising benzalkonium chloride and calcitonin as peptide.

Benzalkonium chloride and benzethonium chloride are both effective at an extremely low concentration, for example, at 0.001% or higher in preventing adsorption of a peptide on the wall of a container. The concentration of the peptide is therefore maintained constant in the composition of this invention. The peptide can therefore be administered in the same amount as it is incorporated upon preparation of the composition.

The above and other objects, features and advantages of this invention will become apparent from the following detailed description of the invention and the appended claims, taken in conjunction with the accompanying drawings.

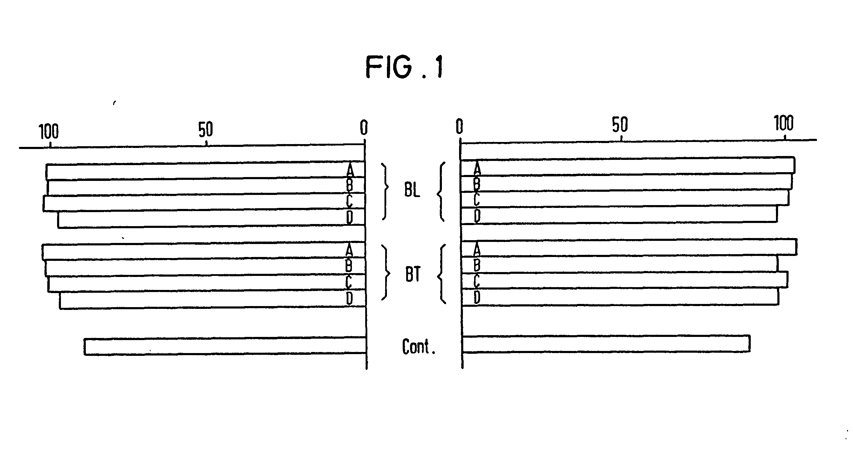

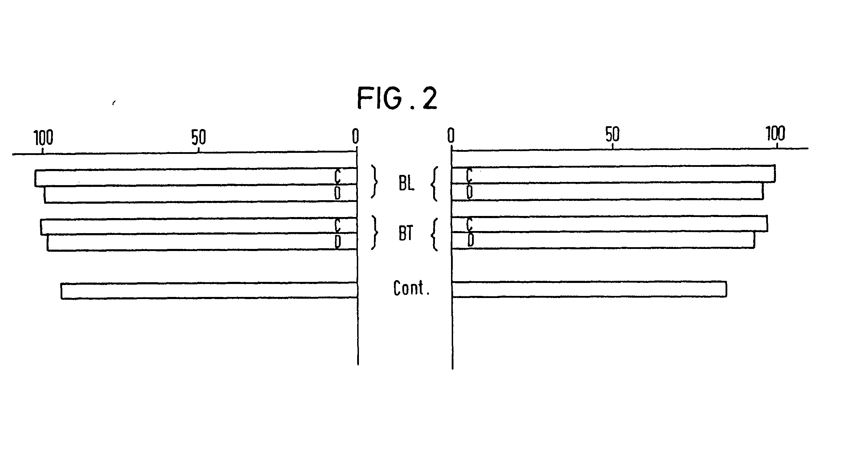

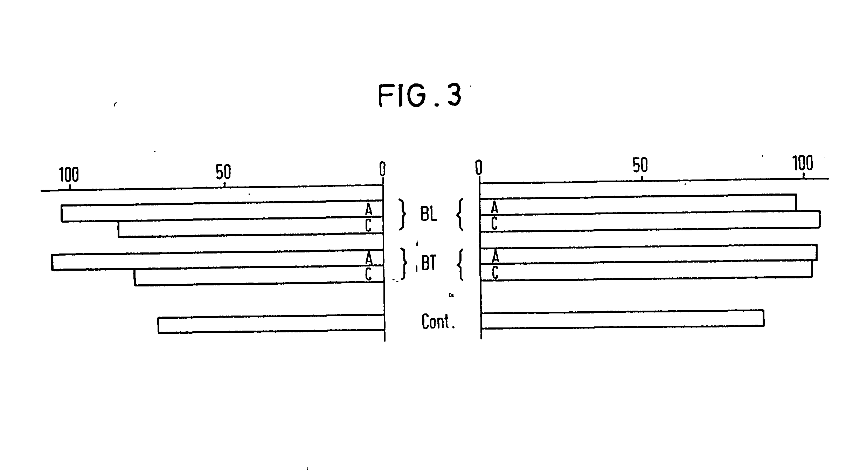

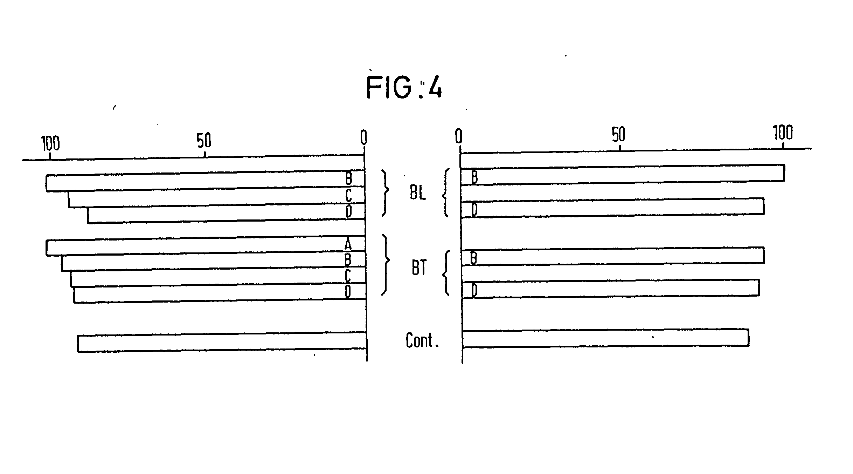

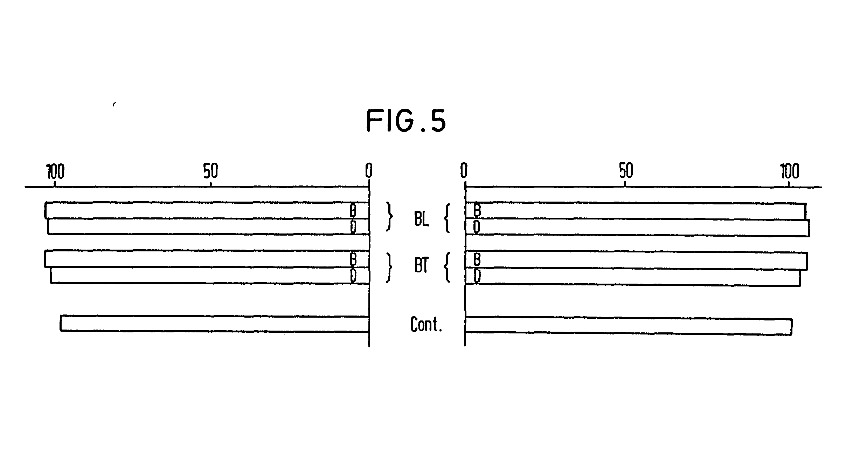

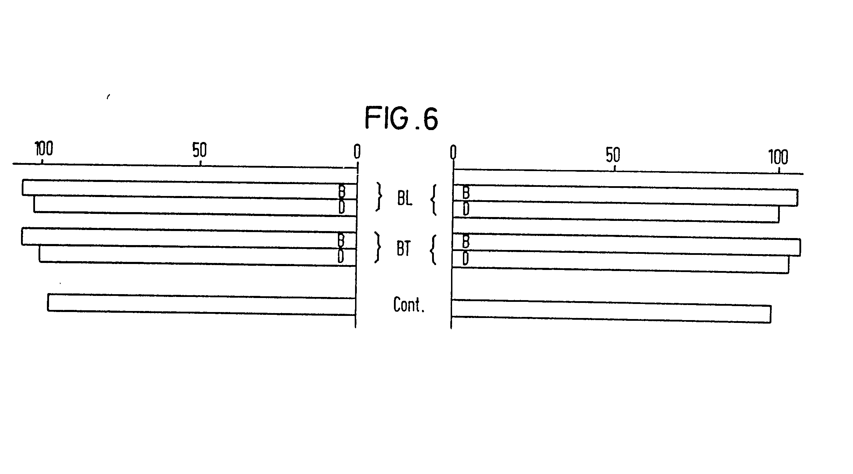

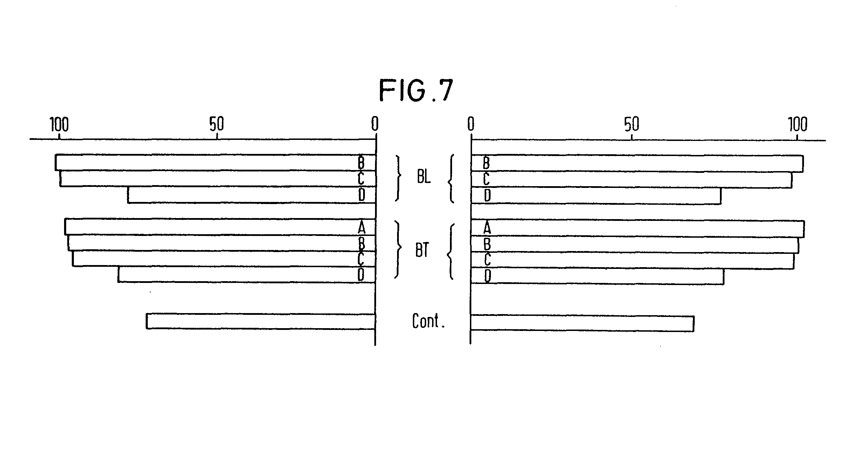

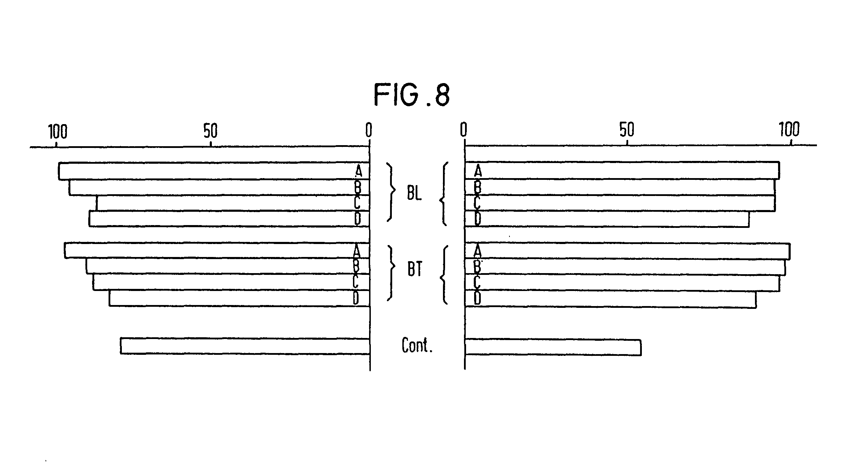

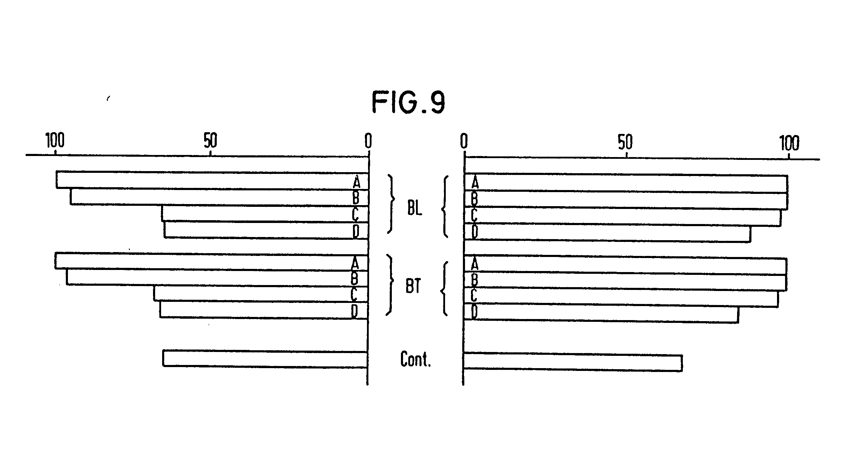

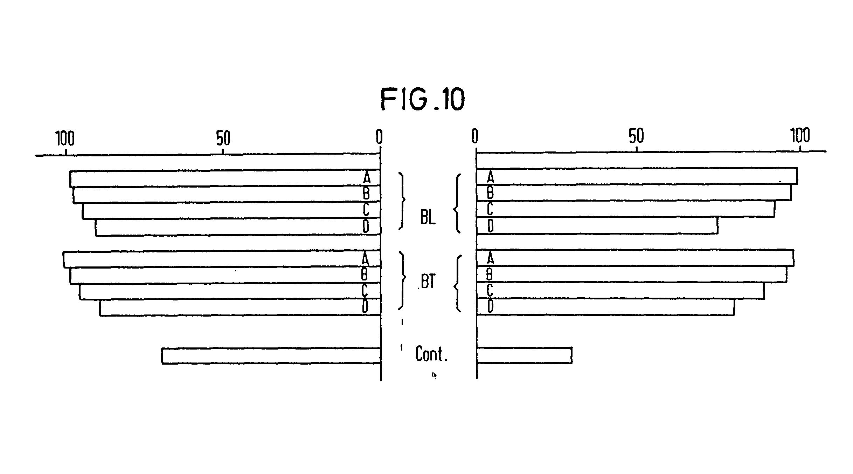

Figs. 1 through 10 are histograms showing the relations between the percentage recovery of various peptides, namely, substance-P (Fig. 1), neutrotensin (Fig. 2), ACTH (Fig. 3), oxytocin (Fig. 4), bradykinin (Fig. 5), neoendorphin (Fig. 6), dynorphin (Fig. 7), angiotesin I (Fig. 8), secretin (Fig. 9) and insulin (Fig. 10) and the type and concentration of an additive added in accordance with this invention. In each of the drawings, the lefthand histogram shows results obtained by using glass bottles while the righthand histogram illustrates results obtained by using plastic bottles.

The term "peptide" as used herein may for example include insulin, secretin, P-substance, angiotensin I, bradykinin, neoendorphin, neurotensin, calcitonin, oxytocin, glucagon, ACTH and dynorphin. The molecular weights of peptides having physiological effects fall generally within a range of from 200 g/mol to 6,000 g/mol in many instances. The molecular weights of the above-exemplified peptides also fall within a range of from 1,000 g/mol to 6,000 g/mol. Since the present invention can exhibit useful preventive effects against all peptides which tend to adsorb on container or equipment walls, the present invention shall not be limited to any particular weight range.

The adsorption-preventive additive is benzalkonium chloride or benzethonium chloride in the present invention as will be demonstrated in Experiments and Examples, which will be described herein.

It was found by the present inventor that these substances can be specifically selected for the prevention of peptide adsorption and they can exhibit almost perfect adsorption-preventive effects at considerably low concentrations. When the peptide-containing composition takes the form of an aqueous solution or suspension, benzalkonium chloride or benzethonium chloride may desirably be contained in an amount of 0.001 or higher in the aqueous solution or suspension. Its concentration may desirably be at such a low level that it does not show any pharmacological effects upon its in-vivo administration. For these reasons, it is preferred to incorporate benzalkonium chloride or benzethonium chloride in such an amount that its concentration is 1% or lower in a resulting aqueous peptide solution or suspension.

The lower concentration limit of the additive in the present invention is not determined by the content of an associated peptide but is governed by the surface area of the inner wall of each container. If the maximum surface area of a container or equipment with which a peptide-containing composition of this invention is brought into contact is known, it is possible to determine, in accordance with the maximum surface area, the amount of the additive to be added to the peptide-containing composition of this invention. In practice, it is however not feasible to know such a surface area in advance. It is hence impossible to determine precisely the content of such an additive in the composition. As will be demonstrated in the below-described Experiments, it is however appropriate in achieving the objects of this invention to control the concentration of the additive within the above-described concentration range, namely, to an amount of 0.001%—1% in an aqueous solution or suspension when the peptide-containing composition takes the form of the aqueous solution or suspension.

The composition of this invention may take several types of preparation forms. It is not absolutely necessary to provide the peptide and the additive of this invention in such a form that they are both contained in the same composition from the beginning. The composition may hence take such a form that the peptide and additive are provided separately and are mixed with each other upon administration of the peptide. The following preparation forms may be mentioned as illustrative embodiments of the composition:

i) A composition in which a peptide and its associated additive of this invention are both contained in the same aqueous solution or suspension.

ii) A kit in which a peptide and its associated additive of this invention take the form of different aqueous solutions or aqueous suspensions. When the peptide is administered, they are mixed with each.

iii) A composition in which a peptide and its associated additive of this invention are both incorporated in the same solid matter or powder. When the peptide is administered, a solution which has been separately furnished is added to the composition to form a solution or suspension.

iv) A kit in which a peptide and its associated additive of this invention are provided as separate solid matters or powders. When the peptide is administeed, they are converted into aqueous solutions or suspensions and are then mixed.

v) A kit in which a peptide is provided as a solid matter or powder while its associated additive of this invention is furnished as an aqueous solution. When the peptide is administered, they are mixed together.

The above-mentioned various aqueous solutions, aqueous suspensions, and solid matters or powders may be readily prepared by usual methods. It is also free to practice in the present invention to add one or more suitable stabilizers, buffer agents and/or the like in such aqueous solutions or aqueous suspensions and to incorporate one or more buffer agents and suitable solidifying or powderizing excipients in such solid matters or powders. In general, benzalkonium chloride and benzethonium chloride are employed singly. They may however be used in combination.

Effects of this invention will hereinafter be described by the following Experiments.

## Experiment 1

(Sample)

Solutions containing benzalkonium chloride respectively at 0.1%, 0.01% and 0.001% in a 0.9% aqueous solution of NaCl and solutions containing benzethonium chloride at the same concentrations in the same aqueous NaCl solution were prepared as sample solutions.

Besides, the 0.9% aqueous solution of NaCl was also provided as a control.

(Method)

The various peptides described in the column entitled "Peptide" in Table 1 were separately dissolved in water to prepare their 50 µg/ml solutions, which were used as neat solutions. The sample solutions were separately poured in an amount of 1 ml per bottle in plastic bottles and glass bottles. The neat peptide solutions were separately added, in an amount of 50 µl per bottle, to the plastic and glass bottles. After vigorously shaking the bottles, 50 µl portions of the resultant mixture were separately taken in microsyringes. They were separately injected in the column of a high-speed liquid chromatography instrument. The amounts of unadsorbed peptides were measured by the high-speed liquid chromatography instrument to determine their recovery rates.

The high-speed liquid chromatography instrument was operated at a measurement wavelength of 200 nm with mobile phases given in Table 1.

Table 1

| Peptide | Mobile phase |
|---|---|
| P-substance | $0.1\text{-}M \ NaClO_4(pH \ 3.0)\text{-}CH_3CN = 65:35$ |
| Angiotensin I | $0.1\text{-}M \ NaClO_4(pH \ 3.0)\text{-}CH_3CN = 67:33$ |
| Bradykinin | $0.1\text{-}M \ NaClO_4(pH \ 3.0)\text{-}CH_3CN = 65:35$ |
| Neoendorphin | $0.1\text{-}M \ NaClO_4(pH \ 3.0)\text{-}CH_3CN = 65:35$ |
| Neurotensin | $0.3\text{-}M \ NaClO_4(pH \ 3.0)\text{-}CH_3CN = 65:35$ |
| Oxytocin | $0.3\text{-}M \ NaClO_4(pH \ 3.0)\text{-}CH_3CN = 75:25$ |
| ACTH | $0.2\text{-}M \ NaClO_4(pH \ 3.0)\text{-}CH_3CN = 60:40$ |
| Dynorphin | $0.3\text{-}M \ NaClO_4(pH \ 3.0)\text{-}CH_3CN = 62:38$ |
| Insulin | $1.4\% \ HClO_4\text{-}CH_3CN = 65:35$ |
| Secretin | $0.2\% \ HClO_4\text{-}CH_3CN = 60:40$ |

Results:

Results are shown in Figs. 1 through 10, which correspond to P-substance, neurotensin, ACTH, oxytocin, bradykinin, neoendorphin, dynorphin, angiotensin I, secretin and insulin respectively. In each drawing, the lefthand histogram shows results obtained by using glass bottles while the righthand histogram illustrates those obtained by using plastic bottles.

In each drawing, "Cont." corresponds to recovery rates obtained from the use of the 0.9% aqueous solution of NaCl only. On the other hand, "BL" and "BT" indicate recovery rates obtained respectively when benzalkonium chloride and benzethonium chloride were added separately to the 0.9% aqueous solution of NaCl.

"A", "B", "C" and "D" indicate recovery rates of the respective peptides when the contents of the associated additives were 0.1%, 0.01%, 0.001 and 0.0001% respectively.

From Figs. 1 through 10, it is clearly envisaged that the additives of this invention serve as adsorption-preventing agents. It is also appreciated that the additives of this invention can each exhibit its effect and can hence prevent peptides from being adsorbed on containers or equipment when incorporated at a concentration of 0.001% or higher.

4

The present invention will hereinafter be described specifically by the following Examples.

## Example 1

An aqueous solution having a total volume of 100 ml and containing calcitonin (16,000 units) and glycin (4.0 g) was aseptically prepared. It was filled 1 ml by 1 ml in vials. The aqueous solution was solidified by lyophilization, followed by their sealing.

A 0.01% aqueous solution of benzethonium chloride was also aseptically prepared. It was filled 1 ml by 1 ml in ampules, followed by their sealing to provide an ampule-filled solution for dissolution.

## Example 2

An aqueous solution having a total volume of 100 ml and containing oxytocin (200 units), benzalkonium chloride (0.10 g) and sodium chloride (0.90 g) was aseptically prepared, followed by its aseptic adjustment to pH 2.1—4.5 with hydrochloric acid. It was then filled 1 ml by 1 ml in ampules, followed by their sealing.

## Example 3

An aqueous solution containing L-alanine (4 g) and benzalkonium chloride (0.01 g) in 0.03 M citrate/disodium phosphate buffer of pH 4.0 (100 ml) was aseptically prepared. It was filled 1 ml by 1 ml in vials, followed by their sealing. Ampules containing injection-grade distilled water (1 ml) were also provided as ampule-filled water for dissolution.

## Example 4

An aqueous solution having a total volume of 100 ml and containing ACTH (2,500 units) and benzethonium chloride (0.10 g) was aseptically prepared. It was filled 1 ml by 1 ml in silicone-coated ampules, followed by their sealing.

## Example 5

An aqueous solution having a total volume of 100 ml and containing ACTH (2,500 units), 0.05 g of benzalkonium (0.05 g) and benzethonium chloride (0.03 g) was aseptically prepared. It was filled 1 ml by 1 ml in ampules, following by their sealing.

## Example 6

A 0.1% aqueous solution of benzalkonium chloride was aseptically prepared. It was filled 2 ml by 2 ml in ampules, followed by their sealing to provide an ampule-filled solution for the prevention of peptide adsorption.

## Example 7

An aqueous solution containing benzalkonium chloride (0.05%) and benzethonium chloride (0.05%) was aseptically prepared. It was filled 2 ml by 2 ml in ampules, followed by their sealing to provide an ampule-filled solution for the prevention of peptide adsorption.

**Claims for the Contracting States: BE CH DE FR GB IT LI NL SE**

1. The use of benzalkonium chloride and/or benzethonium chloride for the inhibition of adsorption of peptides on glass and plastic.

2. An adsorption-resistant peptide composition against glass and plastic comprising benzalkonium chloride and/or benzethonium chloride as an essential component with the exception of a composition comprising benzalkonium chloride and calcitonin as peptide.

3. An adsorption-resistant peptide composition as claimed in claim 2, wherein the peptide is P-substance, angiotensin I, bradykinin, neoendorphin, neurotensin, calcitonin, oxytocin, glucagon, ACTH, dynorphin, secretin or insulin.

4. An adsorption-resistant peptide composition as claimed in claim 2 or 3, wherein the composition takes the form of an aqueous solution or suspension and benzalkonium chloride and/or benzethonium chloride are contained in an amount of 0.001—1% in the aqueous solution or suspension.

**Claims for the Contracting State: AT**

1. Use of benzalkonium and/or benzethonium chloride for the inhibition of adsorption of peptides on glass and plastic.

2. Process for the preparation of an adsorption-resistant peptide composition against glass and plastic characterized in that benzalkonium chloride and/or benzethonium chloride as an essential component are added to the composition, with the exception of the addition of benzalkonium chloride to a composition comprising calcitonin as peptide.

3. Process according to claim 2 in which the peptide is P-substance, angiotensin I, bradykinin, neoendorphin, neurotensin, calcitonin, oxytocin, glucagon, ACTH, dynorphin, secretin or insulin.

# EP 0 199 992 B1

4. Process according to claim 2 or 3 in which the composition is prepared in the form of an aqueous solution or suspension and benzalkonium chloride and/or benzethonium chloride are added in an amount such that their total or final concentration is 0.001 to 1% w/v of the aqueous solution or suspension.

**Patentansprüche für die Vertragsstaaten: BE CH DE FR GB IT LI NL SE**

1. Verwendung von Benzalkonium- und/oder Benzethoniumchlorid zur Inhibierung der Adsorption von Peptiden an Glas und Kunststoff.

2. Adsorptionswiderstandsfähige Peptidzusammensetzung gegenüber Glas und Kunststoff, die Benzalkoniumchlorid und/oder Benzethoniumchlorid als eine Hauptkomponente umfaßt, wobei eine Zusammensetzung, die Benzalkoniumchlorid und Calcitonin als Peptid enthält, ausgenommen ist.

3. Adsorptionswiderstandsfähige Peptidzusammensetzung nach Anspruch 2, wobei das Peptid P-Substanz, Angiotensin I, Bradykinin, Neoendorphin, Neurotensin, Calcitonin, Oxytocin, Glucagon, ACTH, Dynorphin, Secretin oder Insulin ist.

4. Adsorptionswiderstandsfähige Peptidzusammensetzung nach Anspruch 2 oder 3, wobei die Zusammensetzung die Form einer wässrigen Lösung oder Suspension einnimmt und Benzalkoniumchlorid und/oder Benzethoniumchlorid in einer Menge von 0,001 bis 1% in der wässrigen Lösung oder Suspension enthalten sind.

**Patentansprüche für den Vertragsstaat: AT**

1. Verwendung von Benzalkonium- und/oder Benzethoniumchlorid zur Inhibierung der Adsorption von Peptiden an Glas und Kunststoff.

2. Verfahren zur Herstellung einer adsorptionswiderstandsfähigen Peptidzusammensetzung gegenüber Glas und Kunststoff, dadurch gekennzeichnet, daß man Benzalkoniumchlorid und/oder Benzethoniumchlorid als Hauptkomponente zu der Zusammensetzung hinzugibt, wobei die Zugabe von Benzalkoniumchlorid zu einer Zusammensetzung, die Calcitonin als Peptid enthält, ausgenommen ist.

3. Verfahren nach Anspruch 2, dadurch gekennzeichnet, daß das Peptid P-Substanz, Angiotensin I, Bradykinin, Neoendorphin, Neurotensin, Calcitonin, Oxytocin, Glucagon, ACTH, Dynorphin, Secretin oder Insulin ist.

4. Verfahren nach Anspruch 2 oder 3, dadurch gekennzeichnet, daß man die Zusammensetzung in Form einer wässrigen Lösung oder Suspension herstellt und Benzalkoniumchlorid und/oder Benzethoniumchlorid in einer solchen Menge hinzugibt, daß ihre Gesamt- oder Endkonzentration 0,001 bis 1% Gew./Vol. der wässrigen Lösung oder Suspension beträgt.

**Revendications pour les Etats contractants: BE CH DE FR GB IT LI NL SE**

1. Utilisation du chlorure de benzalkonium et/ou du chlorure de benzéthonium pour l'empêchement de l'adsorption de peptides sur le verre et le plastique.

2. Composition de peptide résistant à l'adsorption sur le verre et le plastique, comprenant du chlorure de benzalkonium et/ou du chlorure de benzéthonium en tant que composant(s) essentiel(s), à l'exception d'une composition comprenant du chlorure de benzalkonium et de la calcitonine en tant que peptide.

3. Composition de peptide résistant à l'adsorption selon la revendication 2, dans laquelle le peptide est la substance P, l'angiotensine I, la bradykinine, la néoendorphine, la neurotensine, la calcitonine, l'oxytocine, le glucagon, l'ACTH, la dynorphine, la sécrétine ou l'insuline.

4. Composition de peptide résistant à l'adsorption selon la revendication 2 ou 3, dans laquelle la composition est sous la forme d'une solution ou suspension aqueuse et le chlorure de benzalkonium et/ou le chlorure de benzéthonium est(sont) contenu(s) en une quantité de 0,001 à 1% dans la solution ou suspension aqueuse.

**Revendications pour l'Etat contractant: AT**

1. Utilisation du chlorure de benzalkonium et/ou du chlorure de benzéthonium pour l'empêchement de l'adsorption de peptides sur le verre et le plastique.

2. Procédé pour la préparation d'une composition de peptide résistant à l'adsorption sur le verre et le plastique, caractérisé en ce que l'on ajoute à la composition du chlorure de benzalkonium et/ou du chlorure de benzéthonium en tant que composant(s) essentiel(s), à l'exception de l'addition de chlorure de benzalkonium à une composition comprenant de la calcitonine en tant que peptide.

3. Procédé selon la revendication 2, dans lequel le peptide est la substance P, l'angiotensine I, la bradykinine, la néoendorphine, la neurotensine, la calcitonine, l'oxytocine, le glucagon, l'ACTH, la dynorphine, la sécrétine ou l'insuline.

4. Procédé selon la revendication 2 ou 3, dans lequel la composition est préparée sous la forme d'une solution ou suspension aqueuse et on ajoute du chlorure de benzalkonium et/ou du chlorure de benzéthonium en une quantité telle que leur concentration totale ou finale va de 0,001 à 1% p/v de la solution ou suspension aqueuse.

6

FIG . 1

FIG. 2

FIG.3

EP 0 199 992 B1

FIG : 4

FIG.5

EP 0 199 992 B1

FIG.6

FIG.7

FIG.8

FIG.9

FIG.10

EP 0 199 992 B1